# EUROPEAN PATENT APPLICATION

(11) **EP 4 437 891 A1**
(43) Date of publication of application: **02.10.2024**
(21) Application number: 22898617.0
(22) Date of filing: 24.11.2022
(51) Int. Cl.: A41D 1/06, A41D 13/05

(54) **TROUSERS**

(30) Priority: 26.11.2021 JP 2021192524; 10.08.2022 JP 2022128304
(71) Applicant: Midori Anzen Co., Ltd., Tokyo 150-8455 (JP)
(72) Inventor: SAKO Kagari, Soka-shi, Saitama 340-0034 (JP); YANAI Ryusuke, Soka-shi, Saitama 340-0034 (JP); GOMMORI Hideki, Tokyo 150-8455 (JP); HASEBE Yasuo, Tokyo 150-8455 (JP)
(74) Representative: Calysta NV
(86) International application number: PCT/JP2022/043384
(87) International publication number: WO 2023/095835

(57) **Abstract**

To provide pants capable of protecting the lower back without being noticed and of preventing lower back pain for healthy persons who have not experienced the lower back pain by tightening a general belt, pants (1) include a pants main body (10), and a waist portion (20) provided at an upper end of the pants main body (10) around which a belt (B) is detachably wound. The waist portion (20) is provided at a position surrounding the pelvis of a wearer when the pants are worn and includes a belt core (21) and a dimension adjustment portion which is a rubber belt (30) for adjusting the dimension in the waist direction. The belt core (21) faces at least front and rear sides of the pelvis. The belt core (21) is thicker than the belt (B)and has stiffness enough to transmit the tightening force of the belt (B) to the pelvis.

## Description

### TECHNICAL FIELD

The present invention relates to pants worn by a wearer on a lower body thereof.

### BACKGROUND

Conventionally, lower back protective belts worn around the lower back have been known to prevent or alleviate lower back pain (see, for example, Patent Literature 1 and Patent Literature 2). Also known are pants (trousers or slacks) with the lower back protective belt, in which the lower back protective belt is attached to the waist portion of the pants to prevent the lower back protective belt from sliding against the wearer's body (see, for example, Patent Literature 3).

### CITATION LIST

### Patent Literature

Patent Literature 1: Japanese Patent No. 3597228
Patent Literature 2: Japanese Utility model registration No. 3086242
Patent Literature 3: Japanese Patent Publication No. 2008-81864

### SUMMARY

### Technical Problem

However, the conventional lower back protective belts are wide enough to wrap around the wearer's pelvis and are wider than common belts used to prevent pants or trousers from slipping down. As a result, it is easy to see that the wearer is wearing the lower back protective belt or the pants with the lower back protective belt, which makes the wearer look unattractive.

The present invention has been made considering the above issue and an object of the present invention is to provide pants that can protect the lower back of the wearer without being noticed and can prevent lower back pain by tightening an ordinary belt for healthy people who have not experienced the lower back pain.

### Solution to Problem

To achieve the above object, pants of the present invention include a pants main body; and a waist portion that is provided at an upper end of the pants main body, a belt being detachably wrapped around the waist portion. The waist portion is provided at a position surrounding a pelvis of a wearer when the pants are worn and includes a belt core and a dimensional adjustment portion configured to adjust a dimension in a waist direction. The belt core faces at least front and rear sides of the pelvis. The belt core is thicker than the belt and has stiffness enough to transmit a tightening force of the belt to the pelvis.

### Advantageous Effects

The pants of the present invention can protect the lower back without being noticed. In addition, lower back pain can be prevented by tightening an ordinary belt for healthy people who have not experienced lower back pain.

### BRIEF DESCRIPTION OF DRAWINGS

[FIG. 1] FIG. 1 is a plan view illustrating pants in a first embodiment.
[FIG. 2] FIG. 2 is a side view illustrating the pants in the first embodiment.
[FIG. 3] FIG. 3 is a developed view illustrating an outer main part of the pants in the first embodiment.
[FIG. 4] FIG. 4 is a developed view illustrating an inside main part of the pants in the first embodiment.
[FIG. 5] FIG. 5 is a cross-sectional view along a line A-A in FIG. 3.
[FIG. 6] FIG. 6 is a cross-sectional view along a line B-B in FIG. 3.
[FIG. 7] FIG. 7 is a cross-sectional view along a line C-C in FIG. 3.
[FIG. 8] FIG. 8 is a table showing the measurement results of stiffness of a belt core of the first embodiment and belt cores of first and second comparative examples.
[FIG. 9] FIG. 9 illustrates the transmission direction of the tightening force of the belt through the belt core in the first embodiment.
[FIG. 10] FIG. 10 is a view illustrating attached positions of electrodes in first and second tests.
[FIG. 11] FIG. 11 is a view illustrating a trial condition in the first and second tests.
[FIG. 12] FIG. 12 is a view illustrating an ordinary belt used in the first and second tests.
[FIG. 13] FIG. 13 is a table showing the results of a first verification.
[FIG. 14] FIG. 14 is a table showing the results of a second verification.
[FIG. 15] FIG. 15 is a table showing the results of a third verification.
[FIG. 16] FIG. 16 is a table showing average standardized myopotential volumes for first to fourth conditions in the second test.
[FIG. 17] FIG. 17 is a plan view illustrating pants in a second embodiment.
[FIG. 18] FIG. 18 is a side view illustrating the pants in the second embodiment.
[FIG. 19] FIG. 19 is a developed view illustrating an outer main part of the pants in the second embodiment.
[FIG. 20] FIG. 20 is a developed view illustrating an inside main part of the pants in the second embodiment.
[FIG. 21] FIG. 21 is a cross-sectional view along a line D-D in FIG. 19.
[FIG. 22] FIG. 22 is a developed view illustrating an outer main part of the pants in a first variation.
[FIG. 23] FIG. 23 is a developed view illustrating an inside main part of the pants in the first variation.
[FIG. 24] FIG. 24 is a side view illustrating the pants in a second variation.
[FIG. 25] FIG. 25 is a developed view illustrating an outer main part of the pants in the second variation.
[FIG. 26] FIG. 26 is a developed view illustrating an inside main part of the pants in the second variation.
[FIG. 27] FIG. 27 is a cross-sectional view illustrating a part of the pants according to a third variation in a region opposite the front side of the pelvis.
[FIG. 28] FIG. 28 is a cross-sectional view illustrating a part of the pants according to the third variation in a region opposite the rear side of the pelvis.

### DESCRIPTION OF EMBODIMENTS

Pants or trousers of the present invention will be described based on first and second embodiments illustrated in the drawings. In the following description, terms such as "up and down," "left and right," "front and back," and the like are used based on the wearer of the pants 1.

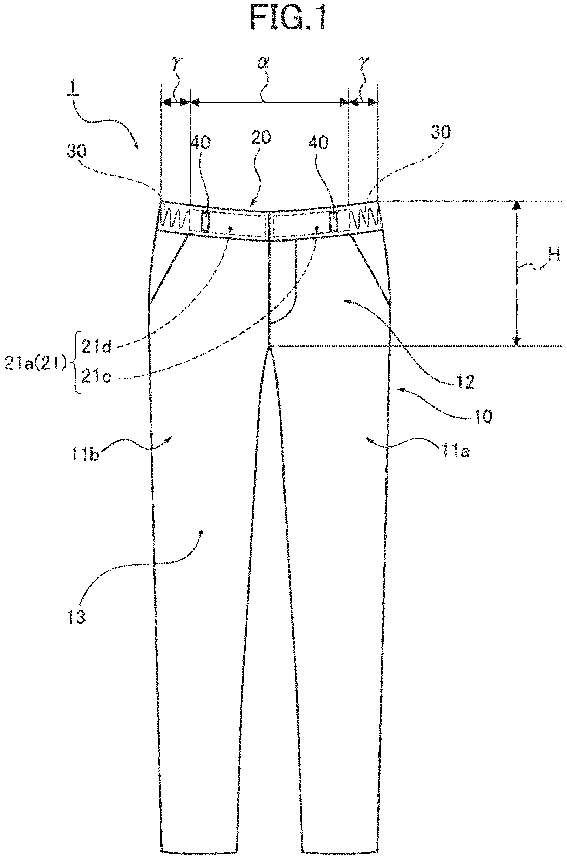

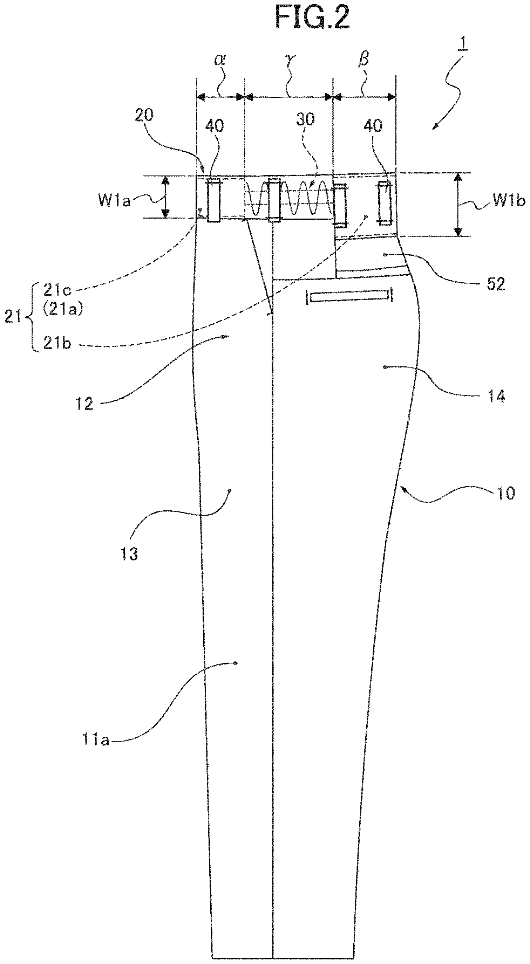

(First Embodiment) As shown in FIGS. 1 and 2, the trousers, slacks, or pants 1 of the first embodiment are long pants worn on the lower half of the wearer's body. The pants 1 are made of fabrics such as woven or non-woven fabrics made of cotton, synthetic fibers, or the like. The fabrics for the pants 1 may have any properties such as elasticity, breathability, flame resistance, water repellency, etc.

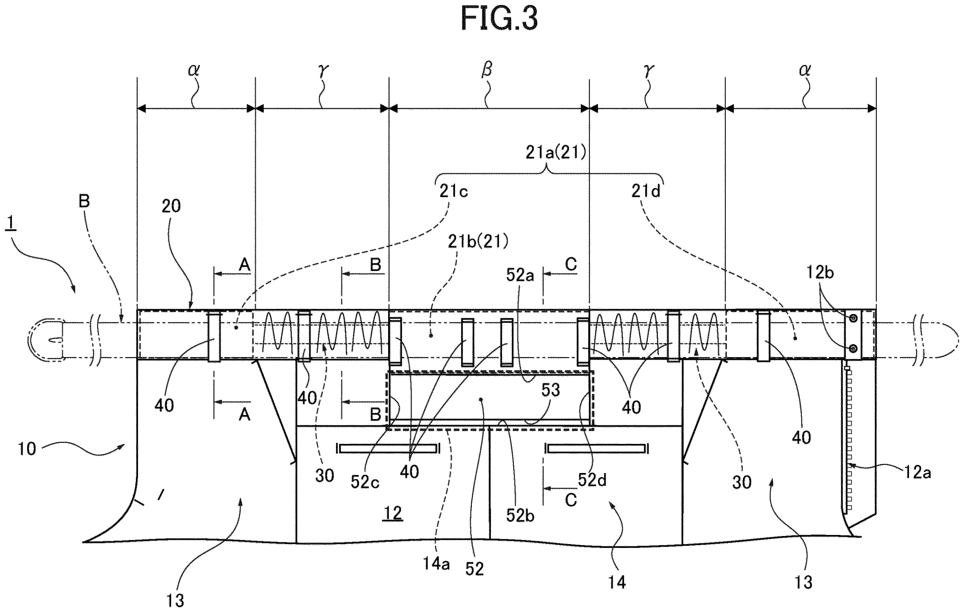

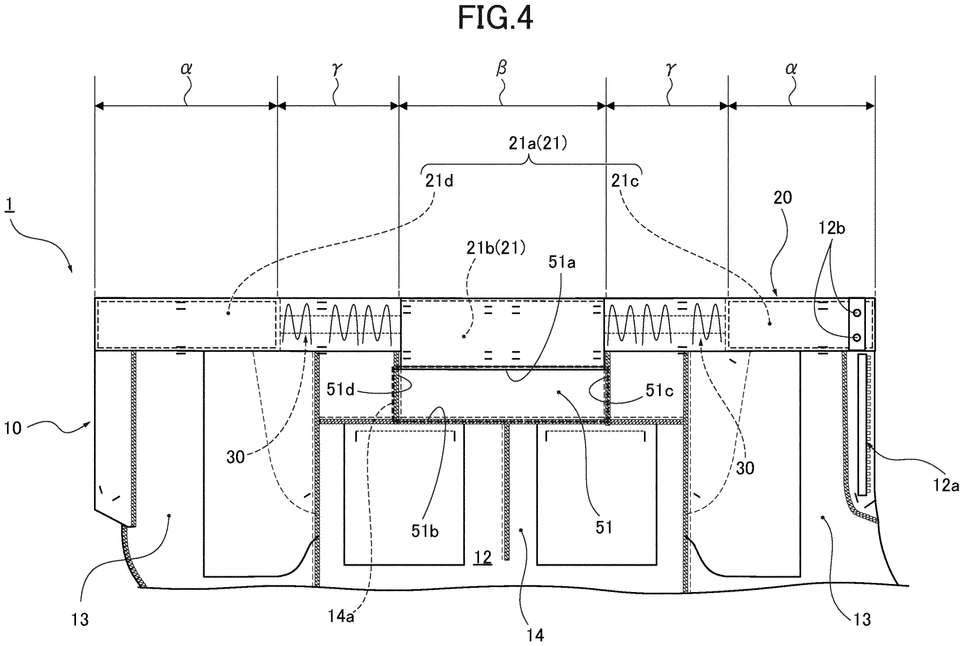

The pants 1 of the first embodiment includes a pants main body 10. The pants main body 10 includes a tubular left leg portion 11a and a tubular right leg portion 11b covering the left and right legs of the wearer, respectively, and a body portion 12 covering the lower abdomen and buttocks of the wearer. The pants main body 10 is divided into left and right sections at the front center of the body portion 12, which can be opened and closed by a liner fastener 12a and/or a snap button 12b (see FIGS. 3 and 4).

The pants main body 10 is made by sewing a front body 13 and a rear body 14 together. The pants main body 10 may be made by three-dimensional sewing with gussets between the front body 13 and the rear body 14. Each of the gussets extend from the hem of the left leg portion 11a through a crotch or inseam portion to the hem of the right leg portion 11b.

The pants main body 10 includes a waist portion 20 at the upper end of the body portion 12. A belt B (see FIG. 3) is detachably wrapped around the waist portion 20 to prevent the pants 1 from slipping down. In the pants main body 10, a length H of the crotch or inseam (i.e., the height of the body portion 12, the portion above the division between the left leg portion 11a and the right leg portion 1 1b) is set to be shallower or shorter than that of ordinary work pants for workers in factories, construction sites, and the like. Accordingly, the waist portion 20 is positioned around the pelvis of the wearer (especially near a portion protruding forward in the pelvis, generally referred to as the "superior anterior iliac spine") when the pants are worn. In other words, the pants main body 10 is designed so that the belt B is positioned around the pelvic position when the wearer wears the pants with the belt B attached.

The belt B attached to the pants 1 in the first embodiment is a general work belt. It is preferable that the belt B has a width of about 3 cm and low elasticity and is adjustable in tightening length using a buckle or the like. The belt B may be, for example, a cloth belt made of polypropylene or nylon, a leather belt, or a rubber belt with high rigidity.

The waist portion 20 includes a belt core 21 and a rubber belt 30.

As shown in FIG. 3, the belt core 21 is provided in an area α facing the front side of the pelvis and an area β facing the rear side of the pelvis. In other words, the belt core 21 includes a first belt core 21a facing the front side of the pelvis and a second belt core 21b facing the rear side of the pelvis. Here, the " area α facing the front side of the pelvis" is an area between the right and left superior anterior iliac spines when the wearer is viewed from the abdominal or front side. The "area β facing the rear side of the pelvis" is an area facing the sacrum of the pelvis when the wearer is viewed from the back or rear side. The first belt core 21a in the area α opposing the front side of the pelvis is further divided into a left-side first belt core 21c and a right-side first belt core 21d since the front side of the body portion 12 is divided into left and right sections (see FIG. 1).

The rubber belt 30 is provided in the area γ (between the front and rear sides of the pelvis) facing the lateral sides of the pelvis. The belt core 21 and the rubber belt 30 do not overlap each other and are alternately positioned along the waist of the wearer since the belt core 21 is provided in the area α facing the front side of the pelvis and the area β facing the rear side of the pelvis, and the rubber belt 30 is provided in the area γ facing the lateral sides of the pelvis.

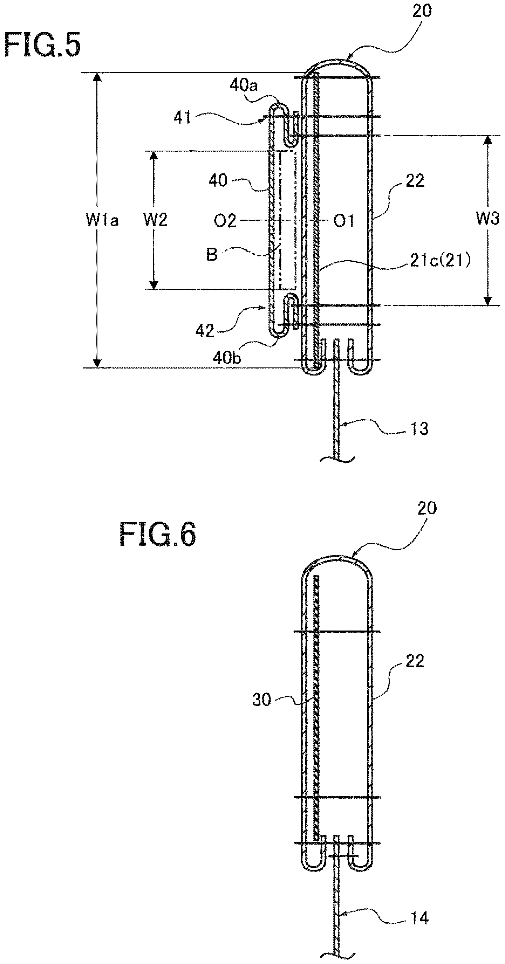

As shown in FIG. 5, a strip belt fabric 22 is folded in half and the first belt core 21a is sandwiched between halves of the belt fabric 22 and attached to the waist portion 20 by sewing the belt fabric 22 to the upper side of the front body 13. The belt fabric 22 is generally made of the same fabric as the fabric of the pants main body 10, but a different fabric may be used. The belt fabric 22 may also include an adhesive core attached to the inside thereof. Similar to the first belt core 21a, the second belt core 21b is also attached to the waist portion 20 by being sandwiched between the halves of the belt fabric 22 and the belt fabric 22 is attached to the waist portion 20 by sewing the belt fabric 22 to the upper side of the rear body 14.

As shown in FIG. 2, in the belt core 21, the width (i.e., length in the vertical direction) W1a of the first belt core 21a in the area α facing the front side of the pelvis is set to be narrower than the width W1b of the second belt core 21b in the area β facing the rear side of the pelvis.

Furthermore, the width W1a of the first belt core 21a is set to be thicker than the width W2 of the belt B (see FIG. 5). In other words, the width of the belt core 21 is set to be thicker than the width W2 of the belt B both in the front and rear sides of the pelvis. It is preferable that the widths W1a, W1b of the first and second belt cores 21a, 21b are set to be about 1.3 to 2.6 times thicker than the width W2 of the belt B. In the first embodiment, the width W1a of the first belt core 21a is set to 6 cm and the width W1b of the second belt core 21b is set to 8 cm while the width W2 of the belt B is 3 cm.

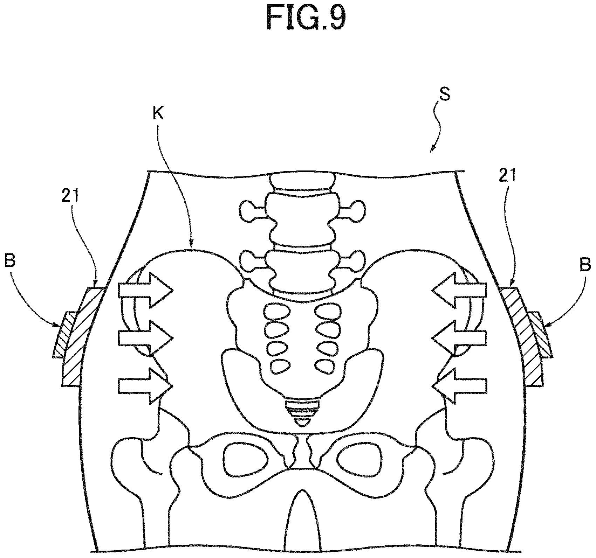

Furthermore, the belt core 21 has stiffness that is higher than the fabrics of the pants main body 10 and the belt fabric 22 and that transmits the tightening force of the belt B to the pelvis (especially near the sacrum). Here, the "stiffness" is an indicator of the flexural or bending rigidity of the belt core 21 (i.e., the stiffness of the belt core 21). "High stiffness" means that the flexural rigidity of the belt core 21 is relatively high, while "low stiffness" means that the flexural rigidity of the belt core 21 is relatively low. The "stiffness that transmits the tightening force of the belt B to the pelvis" means, as shown in FIG. 9, the stiffness that enables the belt core 21 to resist the tightening force of the belt B and prevent the belt B from biting into the wearer S when the pants 1 are worn with the belt B and to transmit the tightening force of the belt B substantially evenly from the entire surface of the belt core 21 to the pelvis K. The "pelvis K" is a skeleton that consists of the right and left hip bones (ilium, pubis, and sit bones), sacrum, and tailbone.

In other words, if the stiffness of the belt core 21 is relatively low, the belt core 21 cannot resist the tightening force of the belt B and bends inward, causing the belt B to bite into the wearer. In this case, the tightening force is concentrated in the area facing the belt B, and the pressure on the pelvis and abdomen becomes too strong. On the other hand, if the stiffness of the belt core 21 is too high, the waist portion 20 becomes hard. As a result, the waist portion 20 does not deform and bites into the abdomen when the wearer is in a sitting posture or a squatting posture, and the waist portion 20 is pinched by the abdomen. Therefore, it is preferable to set the stiffness of the belt core 21 to a degree that allows the tightening force of the belt B to be transmitted to the pelvis and suppresses the biting of the waist portion 20 into the abdomen.

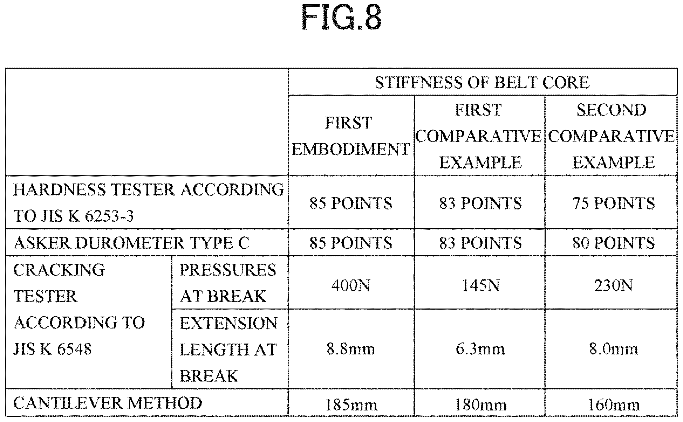

In the first embodiment, the belt core 21 is made of polyester. As shown in FIG. 8, the belt core 21 of the first embodiment has the stiffness of 85 points in both stiffness measurements using a hardness tester according to JIS K 6253-3 and the ASKER Durometer Type C specified in the Standard of the Society of Rubber Industry of Japan (SRIS). The belt core 21 of the first embodiment has stiffness that breaks when the belt core 21 is elongated by 8.8 mm under a 400 N applied pressure using a cracking tester according to JIS K 6548.

Furthermore, the belt core 21 of the first embodiment has the stiffness of 185 mm in the stiffness measurement by the cantilever method. The cantilever method is a stiffness test for cloth. In the cantilever method, a test piece is placed on a horizontal stand having a slope of 45°, the test piece is slid in the direction of the slope, the position when the center point of one end of the test piece touches the slope is read from the scale on the horizontal stand, and the stiffness is evaluated by the length the test piece has moved.

In the stiffness measurements by the hardness tester and cantilever method, the higher the numerical value of the measurement result, the higher the stiffness of the test piece. In the cracking tester, the greater the applied pressure at a break and the longer the extension length at the break, the higher the stiffness of the test piece.

The stiffness of the belt core of the first comparative example and the stiffness of the belt core of the second comparative example used for the general work pants are shown in FIG. 8. In other words, the belt core of the first comparative example has the stiffness of 83 points in both measurements using the hardness tester according to JIS K 6253-3 and the ASCAR hardness tester type C specified in the Standards of the Society of Rubber Industry of Japan (SRIS). The belt core of the first comparative example has the stiffness that breaks when the belt core is elongated by 6.3 mm under a pressure of 145 N when a load is applied using the cracking tester according to JIS K 6548. The belt core of the first comparative example has the stiffness of 180 mm measured by the cantilever method. On the other hand, the belt core of the second comparative example has the stiffness of 75 points when measured with the hardness tester according to JIS K 6253-3, and the stiffness of 80 points when measured with the ASKER Durometer (hardness tester) Type C as specified in the Standard Specifications of the Society of Rubber Industry of Japan (SRIS). The belt core of the second comparative example has the stiffness of 80 points. The belt core of the second comparative example has the stiffness that breaks when it is elongated by 8.0 mm under a pressure of 230 N when a load is applied using a cracking tester according to JIS K 6548. Furthermore, the belt core of the second comparative example has the stiffness that results in a measurement of 160 mm in the stiffness measurement by the cantilever method.

Thus, the belt cores of the first and second comparative examples have stiffness lower (softer) than that of the belt core 21 of the first embodiment. Therefore, for example, when the belt cores of the first and second comparative examples are provided in the waist portion 20, the tightening force of the belt B causes the belt core to flex, resulting in the belt B biting into the wearer, and the pressure on the pelvis and abdomen becomes too strong.

For example, in the case of the waist portion 20 with two layers of the belt core of the second comparative example, the waist portion 20 bites into the abdomen when the wearer is in a sitting or squatting posture. In other words, it was found that the stiffness of the belt core of the second comparative example with two layers of the belt core (the stiffness measured by the cantilever method is about 320 mm) makes the waist portion 20 too hard. Therefore, it is preferable that the stiffness of the belt core 21 of the first embodiment is in the range of 190 mm to 300 mm when measured by the cantilever method.

The rubber belt 30 is a strip or band-shaped rubber cord (i.e., flat rubber). The rubber belt 30 expands and contracts in the longitudinal direction and functions as a dimensional adjustment portion that adjusts the dimension of the waist portion 20 in the waist direction. As shown in FIG. 6, the rubber belt 30 is attached to the waist portion 20 by being sandwiched between the halves of the belt fabric 22 folded in half and sewing the belt fabric 22 to the upper side of the front body 13 or the rear body 14,

The belt fabric 22, which sandwiches the rubber belt 30, is gathered (or has gathers) to follow the elongation of the rubber belt 30. Furthermore, the longitudinal ends of the rubber belt 30 may be sewn to the belt fabric 22 or to the longitudinal ends of the belt core 21.

The pants main body 10 is provided with a plurality of belt loops 40 that holds the belt B at the waist portion 20. The belt loops 40 are strip or band-shaped pieces of fabric extending in the vertical direction. Each of the belt loops 40 includes an upper portion 40a attached to the pants main body 10 by an upper fixing portion 41 and a lower portion 40b attached to the pants main body 10 by a lower fixing portion 42. As shown in FIG. 5, a distance W3 between the upper fixing portion 41 and the lower fixing portion 42 is set to be substantially the same length as the width W2 of the belt B. Moreover, the upper fixing portion 41 and the lower fixing portion 42 are provided at positions so that the center position O2 of the belt B in the width direction coincides with at least the center position O1 of the first belt core 21a in the width direction (see FIG. 5).

Further, the pants main body 10 includes a space 14a in the rear body 14. The space 14a is formed by notching an area of the rear body 14 opposite the back of the wearer's waist (herein, this area has the length of about 1.5 to 3 times the width of the sacrum in the left-to-right direction centered on the wearer's spine (about 30 cm) and about 1.0 to 1.5 times the length of the sacrum in the vertical direction downward from the waist portion 20).

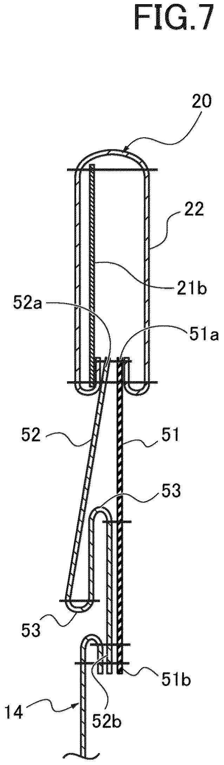

The space 14a is provided with a stretchable fabric 51 and a covering fabric 52, which are layered in order from the inside (wearer's side), as shown in FIG. 7.

The stretchable fabric 51 is a fabric formed from a fabric, such as a power net fabric, which is more stretchable (elastic) than the rear body 14. Generally, the power net fabric is a fine net-like fabric with stretchability and formed by crossing fibers such as nylon and elastic fibers such as polyurethane with each other.

The stretchable fabric 51 is formed in a rectangular shape having an area approximately the same as the space 14a. The stretchable fabric 51 includes a top edge 51a that is sewn between the bottom edges of the belt fabric 22 that sandwiches the second belt core 21b (see FIG. 7). The stretchable fabric 51 also includes a bottom edge 51b and right and left edges 51c, 51d sewn to the rear body 14.

The covering fabric 52 is formed in a rectangular shape. The dimension of the rectangular covering fabric 52 in the left-right direction is approximately equal to the stretchable fabric 51 and the dimension in the vertical direction is larger than the stretchable fabric 51. As shown in FIG. 7, the covering fabric 52 is folded 180° into mountain folds and valley folds, respectively, at a pair of folds or fold lines 53 extending in the left-right direction to form a pleated shape or to have pleats. Folding the covering fabric 52 at the folds 53 makes the vertical dimension of the covering fabric 52 approximately equal to that of the stretchable fabric 51. The covering fabric 52 is made of the same fabric as the rear body 14 and the elasticity of the covering fabric 52 is lower than that of the stretchable fabric 51.

The covering fabric 52 is layered on the outside of the stretchable fabric 51 and includes an upper edge 52a that is sewn together with the stretchable fabric 51 between the lower edges of the belt fabric 22 that sandwiches the belt core 21 (see FIG. 7). The covering fabric 52 also includes a bottom edge 52b, a right edge 52c, and a left edge 52d that are sewn to the rear body 14 together with the stretchable fabric 51.

When the stretchable fabric 51 and the covering fabric 52 are pulled in the vertical direction, the stretchable fabric 51 deforms elastically and the folds 53 of the covering fabric 52 widens, both extending in the vertical direction. When the folds 53 of the covering fabric 52 are extended to the limit, the covering fabric 52 no longer extends in the vertical direction. Therefore, the stretchable fabric 51, which is sewn together with the covering fabric 52, cannot stretch any further, and the stretch of the stretchable fabric 51 is restricted by the covering fabric 52.

The function of the pants 1 in the first embodiment is described below.

To wear the pants 1 of the first embodiment, the wearer first inserts the left leg and the right leg into the left leg portion 11a and right leg portion 11b, respectively, then pulls up the pants main body 10 and closes the front of the body portion 12.

Then, once the wearer has put on the pants main body 10, the wearer puts the belt B through the belt loop 40 and wraps the belt B around the waist portion 20. Finally, the wearer tightens the belt B so that the pants 1 are worn by the wearer with the belt B in place.

In the pants 1 of the first embodiment, the crotch or inseam length H of the pants main body 10 is set shallower or shorter than that of the ordinary work pants, and the waist portion 20 with the belt core 21 is positioned around the pelvis of the wearer. The belt core 21 faces the front and rear sides of the pelvis and has the widths W1a, W1b wider than the width W2 of the belt B. Furthermore, the belt core 21 has stiffness that allows the transmission of the tightening force of the belt B to the pelvis (see FIG. 9).

Therefore, the pants 1 of the first embodiment can tighten the wearer's abdomen by tightening the belt core 21 with the tightening force of the belt B. Therefore, the front and back of the abdomen of the wearer are tightened so that the abdominal pressure is increased and accordingly, the abdominal cavity to function as a pillar to reduce the load on the lower back and increase the protection and reinforcement of the lumbar spine. In addition, as the abdominal pressure increases, the wearer's abdominal muscles are reinforced, and the correct posture can be maintained. Thus, the pants 1 of the first embodiment can protect the lower back of the wearer.

In addition, the pants 1 of the first embodiment tighten the waist portion 20 (belt core 21) by the general belt B, thereby tightening the wearer's abdomen and increasing abdominal pressure. The pants 1 of the first embodiment use the general belt B to increase abdominal pressure, and accordingly, it is difficult to notice from the outside that the waist is protected, thus preventing the deterioration of the appearance of the pants. Thus, the pants 1 in the first embodiment can protect the lower back without being noticed. In addition, the pants 1 of the first embodiment can prevent lower back pain by tightening the general belt B for persons who have not experienced it.

Furthermore, the pants 1 of the first embodiment are provided with the rubber belt 30 in the waist portion 20, which is the dimension adjustment portion for adjusting the dimension in the direction of the waist. Thus, the pants 1 of the first embodiment can stretch and contract the dimension of the waist portion 20 in the direction of the waist according to the wearer's physique, and the belt core 21 can be properly opposed to the front and rear sides of the pelvis of the wearer. This allows the wearer's abdomen to be properly tightened to increase abdominal pressure.

In particular, in the pants 1 of the first embodiment, the dimensional adjustment portion is configured by the rubber belt 30. Therefore, in the pants 1 of the first embodiment, it is possible to change the dimension of the waist portion 20 in the direction of the waist by stretching and contracting the rubber belt 30, so that the dimensional adjustment portion can be formed with a simple configuration. In addition, the rubber belt 30 is sandwiched by the belt fabrics 22 and attached to the waist portion 20. Accordingly, the rubber belt 30 is not visible from the outside of the pants 1. Therefore, in the pants 1 of the first embodiment, it is difficult to recognize that the dimensional adjustment portion (i.e., rubber belt 30 in this embodiment) is provided in the waist portion 20, which prevents the deterioration of the appearance of the pants 1.

Verification tests are described below to verify the effect of reducing the load on the lower back by the pants 1 of the first embodiment. To verify the effectiveness of the pants 1 of the first embodiment in reducing the load on the lower back, the first and second tests were conducted, and the first to third verifications were conducted based on the test results.

The first test was conducted on three adult males (subjects A to C). The second test was conducted on eight adult males (subjects D to K).

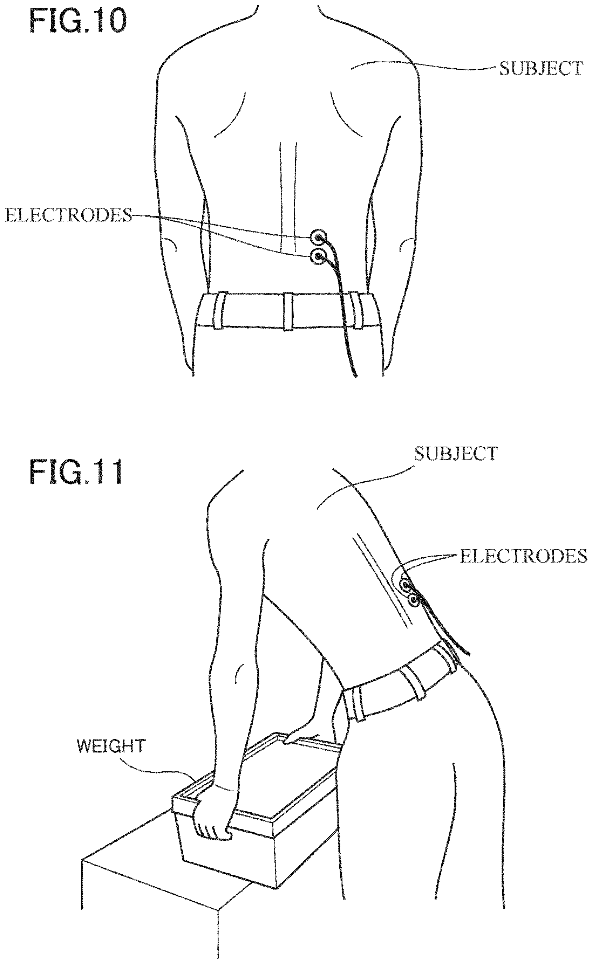

In each of the tests, first, electrodes are attached to the lower back (at positions 5 cm to the right from between the second and third lumbar vertebrae) of each subject (see FIG. 10). Next, in the first test, each of the subjects wears the pants 1 of the first embodiment or the pants of the first comparative example with or without the general belt B. In the second test, each of the subjects wears the pants 1 of the first embodiment or the pants of the second comparative example with or without the belt B. In both tests, each of the subjects then lifts a 20 kg weight placed on a stand at the waist level to a height of approximately 1 cm and holds it for 5 seconds with a forward bend posture of 30° (see FIG. 11). Subsequently, each of the subjects repeats the above trials for three sets with a one-minute break in between, and the myopotential (myogenic potential) amount is measured during each of the trials.

The "pants in the first comparative example" are pants that are normally worn with a special lower back protection belt and have the following requirements.
- The waist portion around which the belt is wrapped is set at a position surrounding the pelvis.
- The belt core faces the front and rear sides of the pelvis.
- The belt core has width narrower than that of the belt.
- The belt core has stiffness lower than that of the belt core of the pants 1 in the first embodiment (the belt core of the first comparative example is used).
- The stretchable fabric is provided at a position opposite the lower back.

The "pants in the second comparative example" are general work pants and have the following requirements.
- The waist area around which the belt is wrapped is set above the pelvis.
- The belt core faces the front and rear sides of the pelvis.
- The belt core has width narrower than that of the belt.
- The belt core has less stiffness than the pants 1 in the first embodiment (using the belt core from the first and second comparative examples).

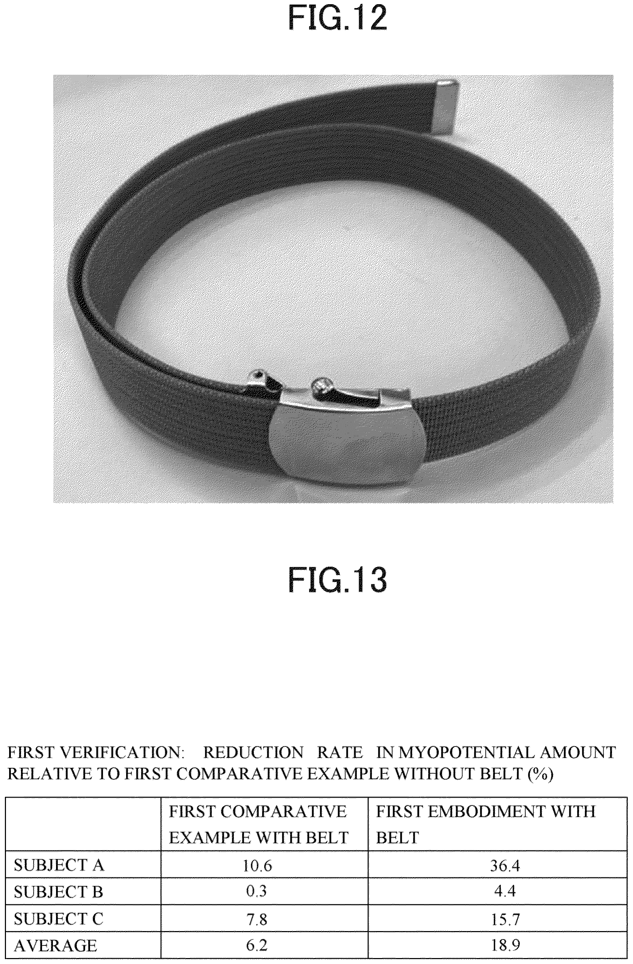

Furthermore, the "belt B" used in each of the tests is a generally used work belt. The belt B herein is a fabric belt made of polypropylene with little or no elasticity and includes a buckle. In each of the tests, the belt B, shown in FIG. 12, was used with the width of approximately 32 mm and the thickness of 2.5 mm. When testing with the belt B, each of the subjects tightened the belt so that the belt length was approximately equal to the wearer's waist dimension (normal tightening), and then tightened the belt further to shorten the belt length by 5%.

In the first verification, the reduction rate of the myopotential amount measured with each subject wearing the pants of the first comparative example with the belt B and the reduction rate of the myopotential amount measured with each subject wearing the pants of the first embodiment with the belt B were calculated for each of the subjects relative to the myopotential amount measured with each subject wearing the pants of the first comparative example without the belt B. The results of the first verification are shown in FIG. 13.

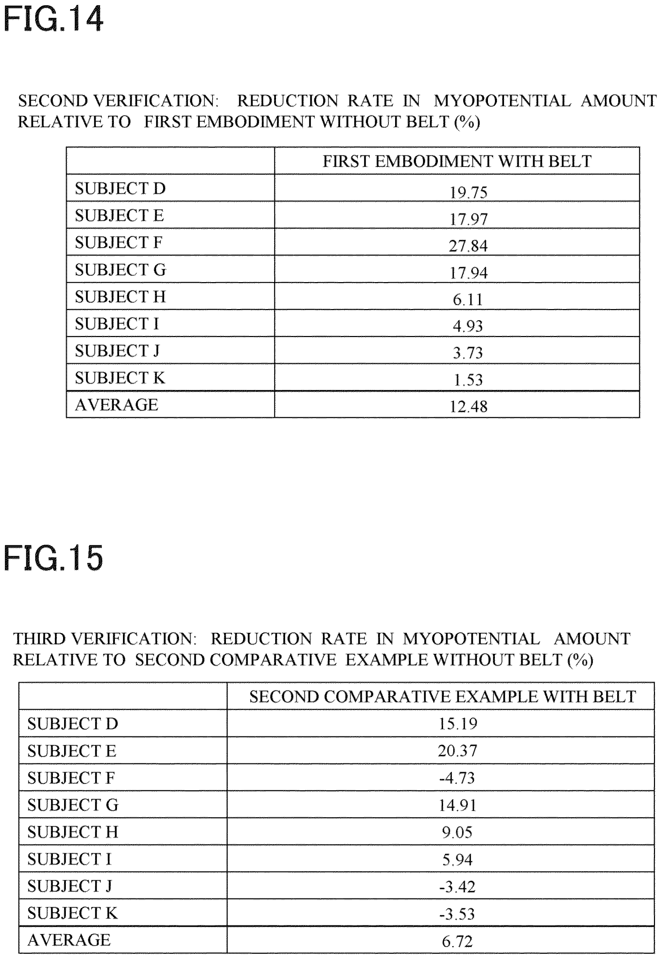

In the second verification, the reduction rate of the myopotential amount measured with each subject wearing the pants of the first embodiment with the belt B was calculated for each of the subjects relative to the myopotential amount measured with each subject wearing the pants of the first embodiment without the belt B. The results of the second verification are shown in FIG. 14.

In the third verification, the reduction rate of the myopotential amount measured with each subject wearing the pants of the second comparative example with the belt B was calculated for each of the subjects relative to the myopotential amount measured with each subject wearing the pants of the second comparative example without the belt B. The results of the third verification are shown in FIG. 15.

The results of the first verification shown in FIG. 13 indicate that in the case of the pants in the first comparative example, the average reduction rate of the myopotential amount was 6.2% and the load on the lower back was reduced even when the general belt B was worn instead of the dedicated lower back protection belt. On the other hand, in the case of the pants 1 in the first embodiment with the belt B, the average reduction rate of the myopotential amount was 18.9%. In short, the results of the first verification indicate that the effect of reducing the load on the lower back when wearing the commonly used work belt B is greater for the pants 1 in the first embodiment than for the pants in the first comparative example.

The results of the second verification shown in FIG. 14 indicate that the average reduction rate of the myopotential amount was 12.48% when the pants 1 of the first embodiment were worn with the belt B attached relative to the case when the pants 1 of the first embodiment were worn without the belt B. In short, the results of the second verification indicate that wearing the pants 1 in the first embodiment with the commonly used work belt B achieves a reducing effect on the load on the lower back.

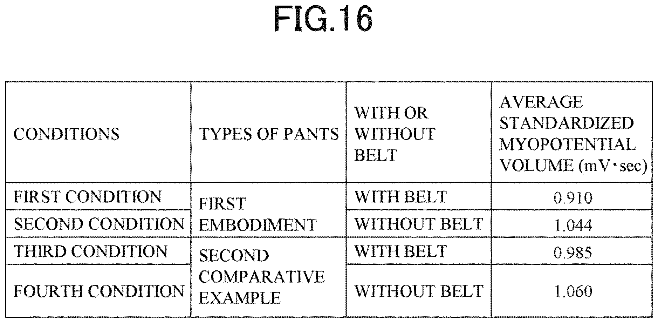

In addition, the statistical analysis was performed based on the results of the average standardized myopotential amount for all subjects in the second test shown in FIG. 16. That is, a one-way analysis of variance (ANOVA) was performed for each of the first to fourth conditions. The results of the one-way ANOVA showed significant differences between the conditions (significance level was less than 5%), and accordingly, multiple comparisons were performed for each of the conditions using Ryan's method. As a result, significant differences were found between the first condition (with the pants 1 in the first embodiment with the belt B) and the second condition (with the pants 1 in the first embodiment without the belt). Thus, the results of the statistical analysis also showed that tightening the belt B significantly reduced the load on the lower back with the pants of the first embodiment.

Furthermore, the results of the third verification shown in FIG. 15 indicate that the average reduction rate of the myopotential amount was 6.72% when the pants of the second comparative example were worn with the belt B attached relative to the case when the pants of the second comparative example, which are the general work pants, were worn without the belt B. In short, the results of the third verification showed that wearing the pants in the second comparative example with belt B also reduced the load on the lower back. However, the multiple comparisons for each of the conditions using Ryan's method showed no significant difference between the third condition (with the pants of the second comparative example with the belt B) and the fourth condition (with the pants of the second comparative example without the belt). Accordingly, the results of the statistical analysis showed that the pants of the second comparative example with the belt B did not significantly reduce the load on the lower back.

On the other hand, when comparing the results of the second and third verifications, it is clear that the average reduction rate of the myopotential amount is higher for the pants 1 of the first embodiment. Accordingly, the results of the second and third verifications indicate that the effect of reducing the load on the lower back with and without the generally used work belt B is greater for the pants 1 of the first embodiment than for the pants of the second comparative example.

Furthermore, during the second test, when the pants of the second comparative example were worn with the belt B, the subjects felt that the abdomen was painful and uncomfortable since the belt dug into the abdomen more than when the pants of the first embodiment were worn with the belt B. From such feelings of the subjects, it was found that the pants 1 of the first embodiment can alleviate the digging of the waist portion 20 into the abdomen compared to the pants of the second comparative example.

Therefore, from the above verification results, it is found that when the pants 1 of the first embodiment are worn and the abdominal pressure is increased by tightening the wearer's abdomen with the tightening force of the commonly used work belt B, the effect of reducing the load on the lower back is greater than when the pants of the first and second comparative examples are worn. In other words, it was found that the pants 1 of the first embodiment had a remarkable reduction effect on the load on the lower back even without using the lower back.

Furthermore, the above effects suggest that by continuously wearing the pants of the first embodiment with the belt B attached, workers who carry heavy loads, care workers, and others can cumulatively reduce the load on the lower back while working.

Moreover, the pants 1 of the first embodiment are provided with a plurality of belt loops 40 holding the belt B to the waist portion 20 in the pants main body 10. The distance W3 between the upper fixing portion 41, which fixes the upper portion 40a of the belt loop 40 to the waist portion 20, and the lower fixing portion 42, which fixes the lower portion 40b of the belt loop 40 to the waist portion 20, is set to be approximately the same width as the width W2 of the belt B. Furthermore, the upper fixing portion 41 and the lower fixing portion 42 are provided at positions where the center position O2 in the width direction of the belt B is at least aligned with the center position O1 in the width direction of the first belt core 21a.

As a result, the pants 1 of the first embodiment can transmit the tightening force of the belt B to the pelvis in the area α opposite to the front side of the pelvis with the center position O1 in the width direction of the first belt core 21a centered. Here, the front side of the pelvis (abdomen) has no bones and is soft, so the belt core 21 is likely to bite into it. By transmitting the tightening force of the belt B with the center position O1 in the width direction of the first belt core 21a centered, the entire surface of the belt core 21 can be used to support the lower back. Therefore, the pants 1 of the first embodiment can stably support the lower back while suppressing the biting of the first belt core 21a into the wearer, thereby improving the protection performance of the lower back.

Also, the pants 1 of the first embodiment include the stretchable fabric 51 having elasticity and the covering fabric 52 covering the stretchable fabric 51 on the lower side of the waist portion 20 of the pants main body 10 and opposite to the back of the wearer's waist.

Therefore, when the skin of the waist or buttocks of the wearer of the pants 1 are stretched in a squatting posture, a sitting posture, or the like, the stretchable fabric 51 and the covering fabric 52 are pulled and stretched in the vertical direction in response to the stretching of the wearer's skin. As a result, the pants 1 of the first embodiment can suppress the waist portion 20 from being pulled downward and prevent the wearer from experiencing discomfort (such as pressure on the abdomen).

In the pants without the stretchable fabric 51 and the covering fabric 52 in the position of the pants main body 10 opposite to the lower back, when the wearer is in a squatting posture, a sitting posture, or the like, the skin of the waist and buttocks of the wearer is stretched and the waist portion 20 is pulled downward, causing the waist portion 20 to dig into the abdomen of the wearer. As a result, the wearer's abdomen is compressed, which compresses the spine and causes lower back pain. However, in the pants 1 of the first embodiment, the stretchable fabric 51 is stretched and the folds 53 of the covering fabric 52 are widened to reduce the pulling of the waist portion 20 downward. Thus, the abdomen of the wearer is not compressed, and lower back pain can be suppressed.

Moreover, in the pants 1 of the first embodiment, the covering fabric 52 is provided outside of the stretchable fabric 51, and accordingly, the stretchable fabric 51 is not visible from the outside, making it difficult to notice the difference in appearance between the pants 1 and the ordinary pants. Therefore, the pants 1 of the first embodiment can protect the lower back without being noticed.

The covering fabric 52 may not be provided. The pants 1 becomes more breathable and the elasticity of the stretchable fabric 51 is increased by not providing the covering fabric 52.

Furthermore, in the pants 1 of the first embodiment, the width W1a of the first belt core 21a provided in the area α facing the front side of the pelvis is set to be narrower than the width W1b of the second belt core 21b provided in the area β facing the rear side of the pelvis.

Therefore, the pants 1 of the first embodiment can prevent the first belt core 21a from digging into the abdomen when the wearer is in a squatting posture, a sitting posture, or the like and prevent the wearer from experiencing discomfort (such as pressure on the abdomen).

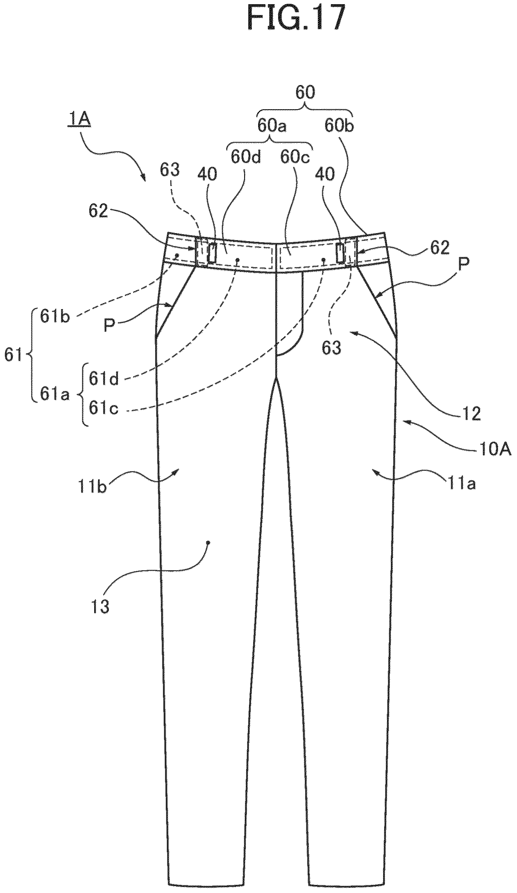

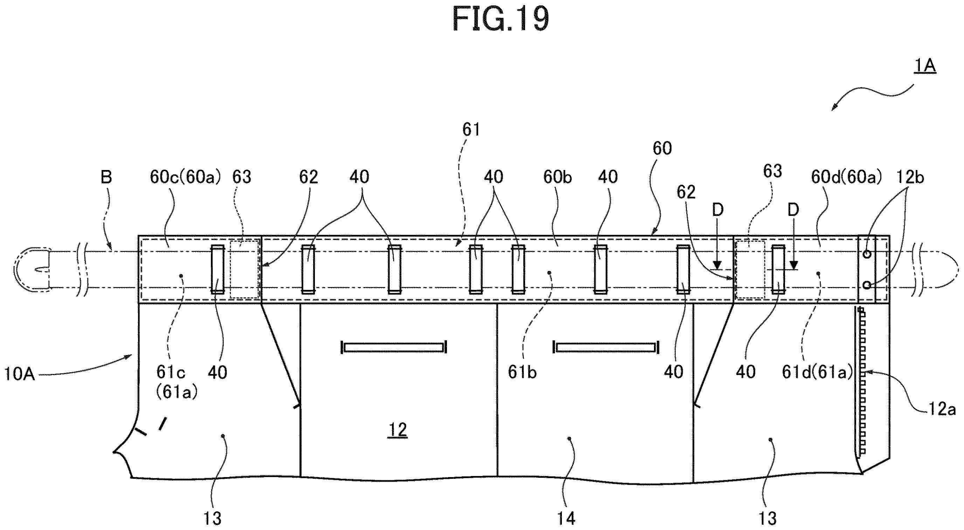

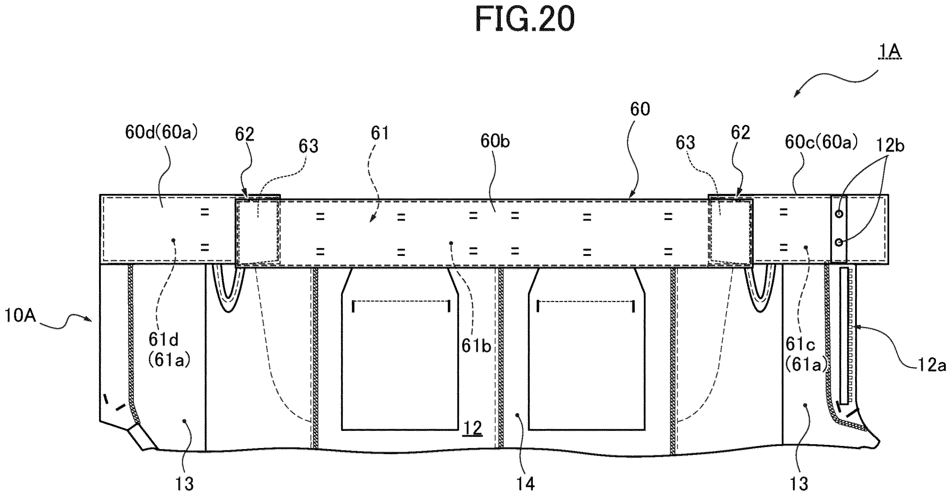

(Second Embodiment) In pants 1A of the second embodiment, as shown in FIGS. 17 to 20, a belt core 61 is provided in a waist portion 60 of a pants main body 10A to surround the entire circumference of the pelvis of the wearer. In the pants 1A of the second embodiment, the dimensional adjustment portion is provided in the waist portion 60 to adjust the dimension of the waist portion 60 in the waist direction. The dimensional adjustment portion consists of a slit 62 that divides the waist portion 60 together with the belt core 61 and an elastic member 63 having elasticity that connects the slit 62.

In other words, the slit 62, which constitutes the dimensional adjustment portion of the second embodiment, is a cutout that extends downward from the upper edge of the pants main body 10A and includes a lower end that is a free end and an upper end that is an end of opening (opening stop) (in this case, at the lower end of a waist pocket P). The slit 62 divides the waist portion 60 together with the belt core 61.

Here, the slit 62 is formed on each side of the pants as shown in FIG. 17. Thus, the slits 62 divide the waist portion 60 into a front waist portion 60a which faces the abdomen of the wearer, and a rear waist portion 60b which faces the flanks and back of the wearer.

The belt core 61 surrounds the entire circumference of the wearer's pelvis and is divided by the slit 62 into a front core 61a facing the abdomen of the wearer and a rear core 61b facing the flanks and back of the wearer. The front core 61a is provided in the front waist portion 60a, and the rear core 61b is provided in the rear waist portion 60b. Furthermore, the front waist portion 60a is further divided into the left front waist portion 60c and the right front waist portion 60d by the front of the body portion 12 is divided into left and right sides. The front core 61a is also divided into a left front core 61c and a right front core 61d.

In the pants 1A of the second embodiment, the width of the front core 61a is set to be the same as that of the rear core 61b. In other words, the belt core 61 of the second embodiment has constant width throughout the entire circumference surrounding the pelvis of the wearer.

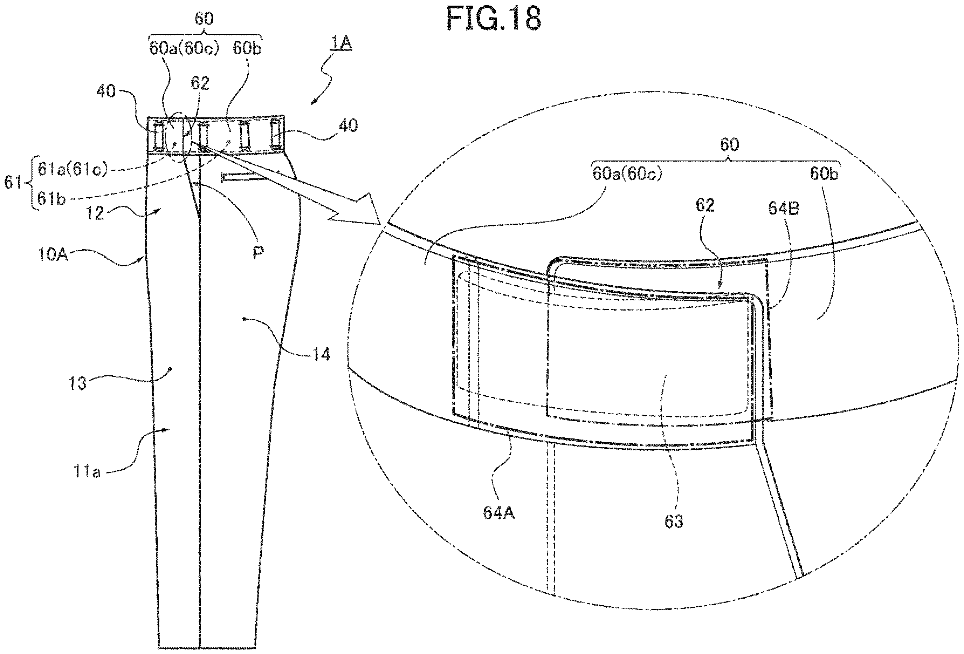

The slit 62 is connected to the stretchable elastic member 63 as shown enlarged in FIG. 18. The elastic member 31 is a flat elastic or rubber having approximately the same width as that of the belt core 21. In the pants 1A of the second embodiment, both sides of the portions of the waist portion 60 divided by the slit 62 (i.e., the rear side area 64A of the front waist portion 60a and the front side area 64B of the rear waist portion 60b) overlap each other as shown in FIG. 18. In other words, the rear side area 64A of the front waist portion 60a is overlaid on the front side area 64B of the rear waist portion 60b.

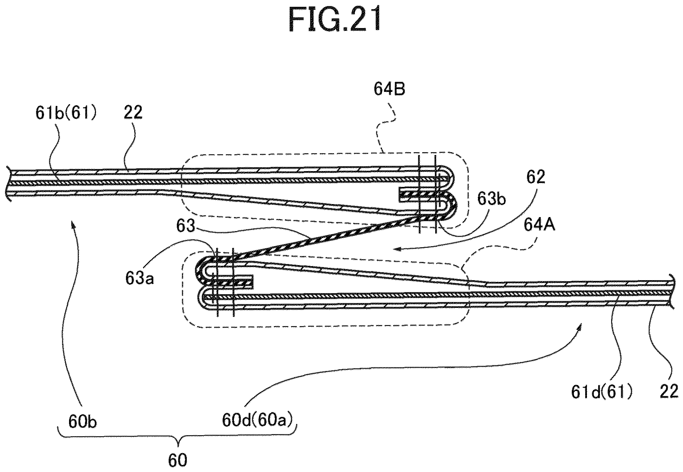

As shown in FIG. 21, the elastic member 63 includes a first end 63a sewn to the rear side area 64A of the front waist portion 60a and a second end 63b sewn to the front side area 64B of the rear waist portion 60b. In other words, the elastic member 63 is provided between the overlapped portions of the waist portion 60 (the rear side area 64A of the front waist portion 60a and the front side area 64B of the rear waist portion 60b).

The pants 1A of the second embodiment are configured without the stretchable fabric 51 and the covering fabric 52 in the rear body 14.

The function of the pants 1A in the second embodiment is described below.

In the pants 1A of the second embodiment, the dimensional adjustment portion for adjusting the dimension of the waist portion 60 in the waist direction consists of the slit 62 that divides the waist portion 60 together with the belt core 61, and an elastic member 63 having elasticity and connects the slit 62.

Therefore, with the pants 1A of the second embodiment, the elastic member 63 can stretch and contract to adjust the dimension of the waist portion 60 in the waist direction to the wearer's physique, and the belt core 61 can be wrinkled to suppress a strong force acting partially on the wearer, thereby suppressing discomfort to the wearer. In addition, in the pants 2A of the second embodiment, the belt core 61 can be provided to surround the entire circumference of the pelvis, so that the belt B can appropriately tighten the entire circumference of the abdomen of the wearer, increasing the abdominal pressure and effectively protecting the lower back.

In the pants 1A of the second embodiment, the slit 62 is provided on each side of the wearer, and the slits 62 divide the belt core 61 into the front core 61a facing the abdomen of the wearer and the rear core 61b facing the flank and the back of the wearer. Thus, the pants 1A make the slits 62 unnoticeable when worn, and further suppress discomfort in appearance.

Furthermore, in the pants 1A of the second embodiment, both sides of the portions of the waist portion 60 divided by the slit 62 (i.e., the rear side area 64A of the front waist portion 60a and the front side area 64B of the rear waist portion 60b) overlap each other. The elastic member 63 is provided between the overlapping portions of the waist portion 60 (i.e., the rear side area 64A of the front waist portion 60A and the front side area 64B of the rear waist portion 60B).

As a result, the pants 1A in the second embodiment can make the slits 62 and the elastic member 63 even less noticeable than in the case where the waist portion 60 has no overlapping portions, thereby further suppressing discomfort in appearance.

The pants of the present invention have been described based on the first and second embodiments. However, the specific configurations of the pants are not limited to the first and second embodiments, and design changes, additions, and the like are allowed without departing from the gist of the present invention recited in the attached claims.

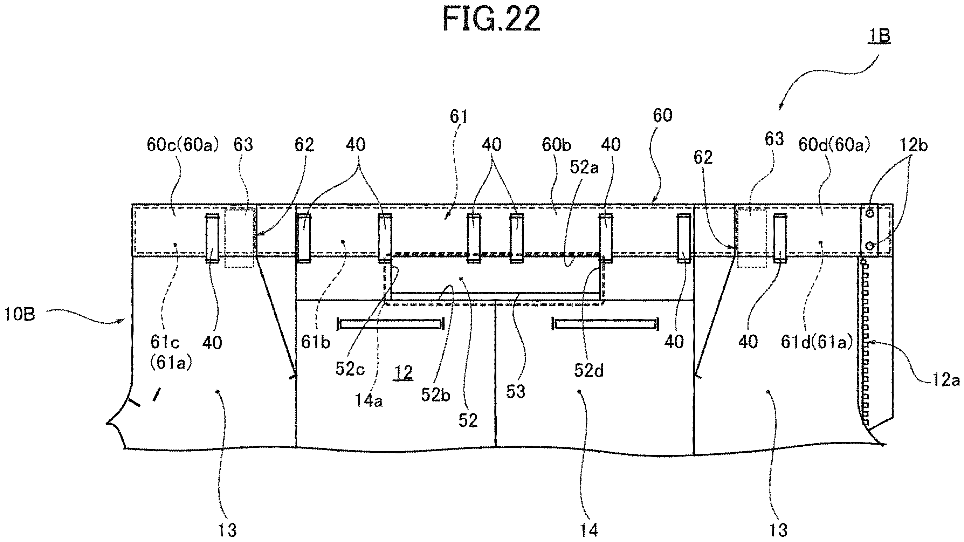

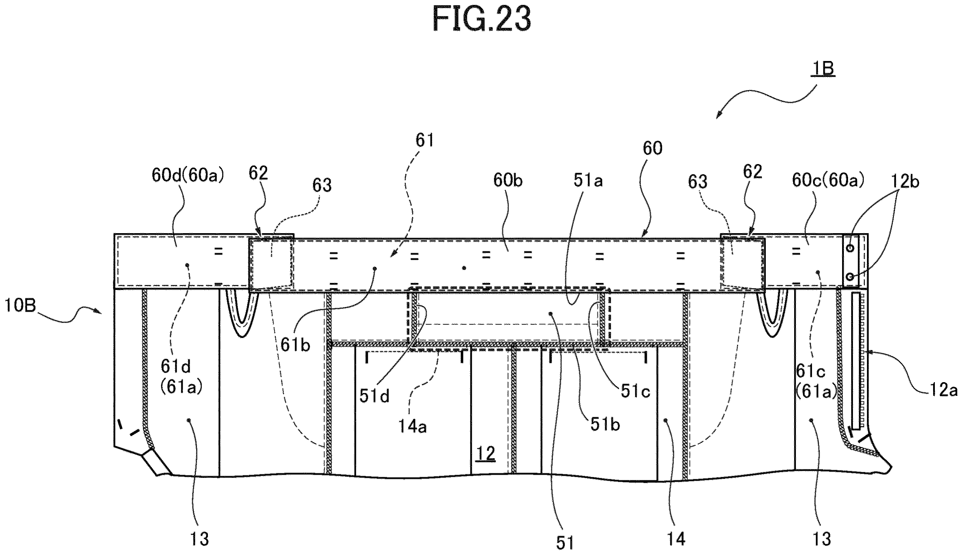

In the pants 1A of the second embodiment, the rear body 14 is not provided with a stretchable fabric 51 having elasticity. However, as in a first variation of pants 1B shown in FIGS. 22 and 23, even when the dimensional adjustment portion consists of the slit 62 and the elastic member 63, the stretchable fabric 51 having elasticity and the covering fabric 52 covering the stretchable fabric 51 may be provided at a position (the rear body 14) below the waist portion 60 and opposite to the back of the wearer's waist,

In other words, in the first variation of the pants 1B, the space 14a is formed in the rear body 14, and the space 14a is provided with the stretchable fabric 51 and the covering fabric 52, which are layered in order from the inside (wearer's side), as shown in FIGS. 22 and 23.

In this first variation of the pants 1B, the belt core 61 is provided around the entire circumference of the pelvis so that the abdomen of the wearer can be appropriately tightened, and the waist portion 60 can be adjusted to the physique of the wearer without causing wrinkles in the belt core 61. Furthermore, in the first variation of the pants 1B, when the wearer is in a sitting posture, a squatting posture, or the like and the skin of the waist or buttocks of the wearer is stretched, the stretchable fabric 51 is also stretched, and the folds 53 of the covering fabric 52 are widened, thereby preventing the waist portion 60 from being pulled downward.

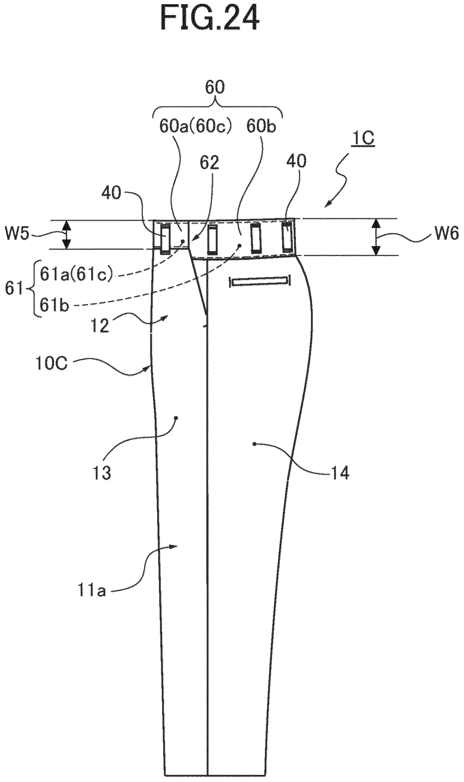

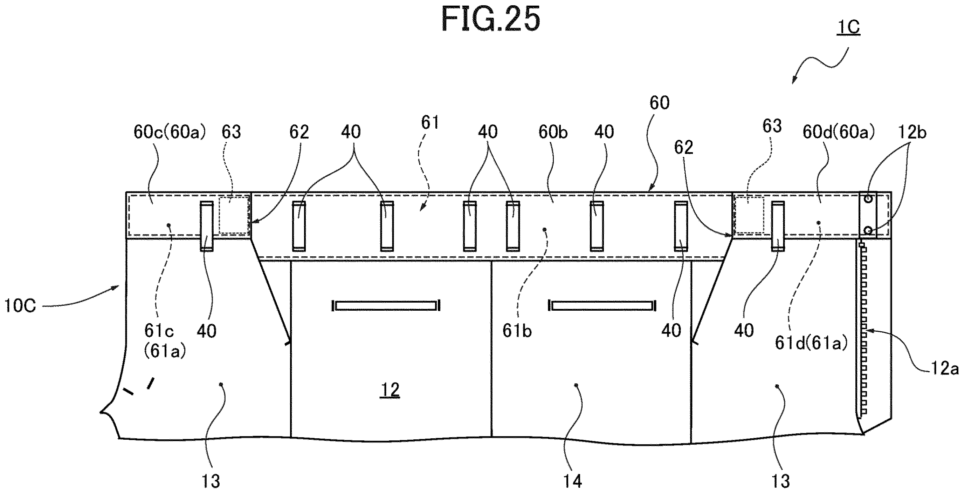

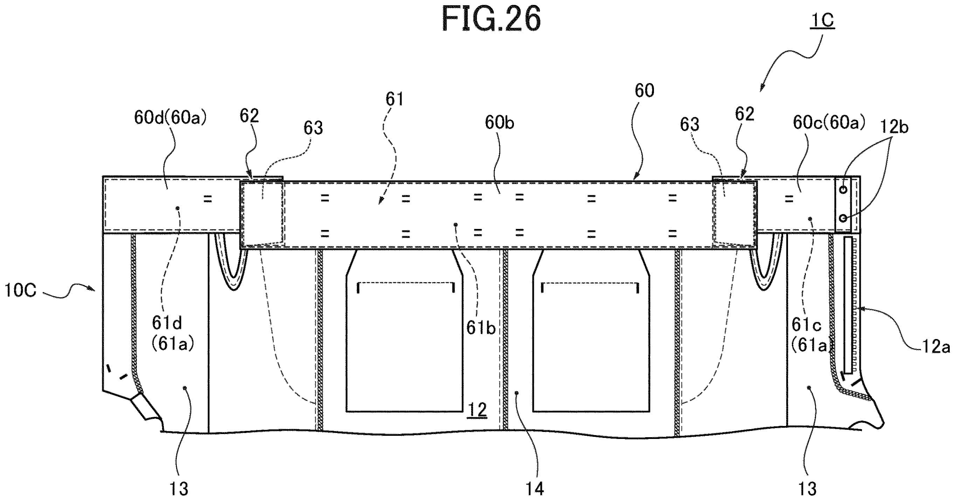

In the pants 1A of the second embodiment, the width of the front core 61a is the same size as that of the rear core 61b, so that the belt core 61 has a constant width throughout the entire circumference surrounding the pelvis of the wearer. However, as in a second variation of pants 1C shown in FIGS. 24 to 26, the width W5 of the front core 61a may be set to be narrower than the width W6 of the rear core 61b even when the dimension adjustment portion consists of the slit 62 and the elastic member 63.

In this second variation of the pants 1C, the belt core 61 is provided to surround the entire circumference of the pelvis, so that the abdomen of the wearer can be appropriately tightened, and the waist portion 60 can be adjusted to the physique of the wearer in the waist direction without causing wrinkles in the belt core 61. Furthermore, the pants 1C of the second variation can stably support the lower back of the wearer while reducing the penetration of the front core 61a into the abdomen of the wearer.

In the first embodiment, the distance W3 between the upper fixing portion 41 and the lower fixing portion 42 of the belt loop 40 is set to be approximately the same size as the width W2 of the belt B to prevent the general pants 1 from slipping down. However, the belt loop 40 is not limited to that shown in the first embodiment. For example, belt loops that are wider than the general belt B and that allow the lower back protection belt to pass therethrough to protect the lower back may be provided in the pants main body 10.

This allows the wearer to wear the lower back protection belt attached to the pants main body 10 according to the wearer's needs. With the lower back protection belt passing through the belt loops, it is possible to prevent misalignment between the pants main body 10 and the lower back protection belt and to maintain the lower back protection belt wrapped around the appropriate position, regardless of the posture of the wearer.

Furthermore, the pants 1 may be provided with a plurality of snap buttons in the waist portion 20 that are aligned vertically and fasten corresponding snap buttons provided on the lower back protection belt. In this case, the pants 1 can adjust and hold the position in which the lower back protection belt is wrapped around the waist, depending on the position in which the snap buttons on the lower back protection belt are fastened.

In the first embodiment, the rubber belt 30 constituting the dimensional adjustment portion is formed on each side of the wearer. In the second embodiment and the first and second variations, the slit 62 and the elastic member 63 constituting the dimensional adjustment portion are formed on each side of the wearer. However, the dimensional adjustment portion may be placed in any position. In addition, the pants main body 10 to 10C may include one or more, for example, three or more dimensional adjustment portions.

Furthermore, the dimensional adjustment portion is not limited to those configured by the rubber belt 30, the slit 62, and the elastic member 63. Since the dimensional adjustment portion is only required to adjust the dimension of the waist portions 20, 60 in the waist direction, the dimensional adjustment portion may be configured by, for example, one or more slits, one or more buttons, and one or more buttonholes, or one or more slits and one or more hooks and one or more hook receptacles. Furthermore, the dimensional adjustment portion may be configured to adjust the waist direction dimension of the waist portion by folding and fastening (holding) the excess waist portion according to the wearer's physique, without a slit.

In the first embodiment, the front side center of the body portion 12 of the pants main body 10 is divided into right and left sides, and the first belt core 21a of the belt core 21 is divided into the left-side first belt core 21c and the right-side first belt core 21d. However, the body portion 12 does not necessarily have to be divided into left and right sides on the front side. The pants main body10 may be worn by widening the rubber belt 30, which is the dimensional adjustment portion, as in the case of so-called all-rubber type pants.

In the pants 1 of the first embodiment, the plurality of belt loops 40 in the pants main body 10 are provided at the position where the center position O2 of the belt B in the width direction coincides with the center position O1 of the first belt core 21a in the width direction. However, the position of the belt loop 40 is not limited to the above example. The upper fixing portion 41 and the lower fixing portion 42 of the belt loop 40 may be provided at positions where the center position O2 of the belt B in the width direction held by the belt loops 40 is shifted in the vertical direction relative to the center position O1 of the first belt core 21a in the width direction.

In the pants 1A of the second embodiment, both sides of the portions divided by the slit 62 of the waist portion 60 (the rear side area 64A of the front waist portion 60A and front side area 64B of the rear waist portion 60B) are overlapped. However, both sides of the portions divided by the slit 62 of the waist portion 60 need not necessarily overlap. Both sides of the slit 62 (the rear side area 64A of the front waist portion 60A and the front side area 64B of the rear waist portion 60B) may be abutted or apart from each other.

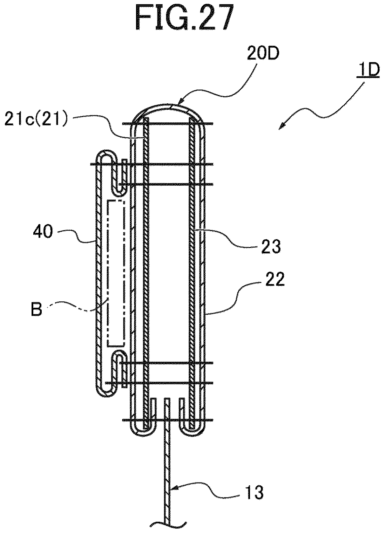

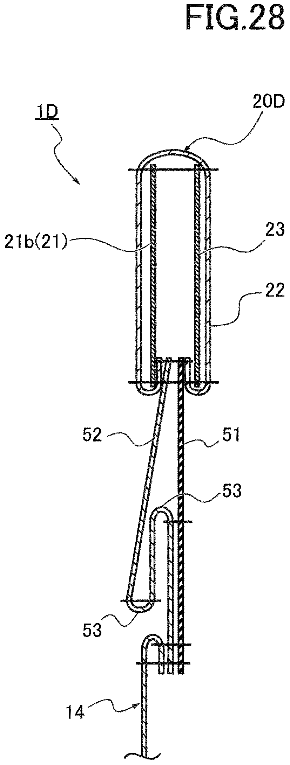

Furthermore, in the first embodiment, the belt core 21 is provided in the area α of the waist portion 20 facing the front side of the pelvis and the area β of the waist portion 20 facing the rear side of the pelvis. However, as in the third variation of the pants 1D shown in FIGS. 27 and 28, a punch core 23 may be disposed inside (on the wearer's side) of the belt core 21 in an area of the waist portion 20D where the belt core 21 is provided (the area α facing the front side of the pelvis and the area β facing the rear side of the pelvis). The punch core 23 is an auxiliary core material that has less stiffness and is softer than the belt core 21. The punch core 23 is made of materials such as a nonwoven fabric. With the punch core 23 disposed inside the belt core 21, the punch core 23 can be a cushioning material and the pants 1D of the third variation can soften the contact of the belt core 21 with the wearer's skin.

### CROSS-REFERENCING OF RELATED APPLICATIONS

The present application is based upon and claims the benefit of priority from Japanese patent application No. 2021-192524 filed with the Japan Patent Office on November 26, 2021, and Japanese patent application No. 2022-128304 filed with the Japan Patent Office on August 10, 2022, the entire disclosure of which is incorporated herein by reference.

## Claims

1. Pants comprising:
a pants main body; and
a waist portion that is provided at an upper end of the pants main body, a belt being detachably wrapped around the waist portion,
wherein the waist portion is provided at a position surrounding a pelvis of a wearer when the pants are worn, and comprises a belt core and a dimensional adjustment portion that is configured to adjust a dimension in a waist direction,
wherein the belt core faces at least front and rear sides of the pelvis, and
wherein the belt core is thicker than the belt and has stiffness enough to transmit a tightening force of the belt to the pelvis.

2. The pants according to claim 1, wherein the dimensional adjustment portion comprises a stretchable rubber belt.

3. The pants according to claim 1, wherein the dimensional adjustment portion comprises:
a slit that divides the waist portion with the belt core; and
an elastic member having elasticity that connects the slit.

4. The pants according to claim 3,
wherein the slit is formed on each side of the wearer, and
wherein the slit is configured to divide the belt core into a front side core facing an abdomen of the wearer and a rear side core facing a back of the wearer.

5. The pants according to claim 3 or 4,
wherein both sides of portions of the waist portion divided by the slit overlap each other, and
wherein the elastic member is provided between the portions overlapping each other.

6. The pants according to one of claims 1 to 5,
wherein the pants main body comprises belt loops that are configured to hold the belt at the waist portion,
wherein each of the belt loops comprises an upper fixing portion that is configured to fix an upper part of the belt loop to the pants main body, and a lower fixing portion that is configured to fix a lower part of the belt loop to the pants main body, and a distance between the upper fixing portion and the lower fixing portion is set to be approximately same as a width of the belt, and
wherein the upper fixing portion and the lower fixing portion are provided at positions where a center position of the belt in a width direction coincides with a center position of the belt core in the width direction.

7. The pants according to one of claims 1 to 6, wherein the pants main body comprises a stretchable fabric at a position below the waist portion and opposite to a lower back of the wearer.

8. The pants according to one of claims 1 to 7, wherein a width of the belt is narrower in an area facing the front side of the pelvis than in an area facing the rear side of the pelvis.
